# EUROPEAN PATENT APPLICATION

(11) **EP 4 579 284 A1**
(43) Date of publication of application: **02.07.2025**
(21) Application number: 23220222.6
(22) Date of filing: 26.12.2023
(51) Int. Cl.: G01T 1/14, A61N 5/10, G01T 1/16

(54) **HYBRID 2D-DETECTOR**

(71) Applicant: Ion Beam Applications, 1348 Louvain-la-Neuve (BE)
(72) Inventor: ROSSOMME, Séverine, 1348 Louvain-la-Neuve (BE); DE BECO, Victor, 1348 Louvain-la-Neuve (BE); SOURIS, Kevin, 1348 Louvain-la-Neuve (BE); OSTERRIETH, Cédric, 1348 louvain-la-Neuve (BE)
(74) Representative: Connor, Marco Tom

(57) **Abstract**

The invention relates to a detector (1) for characterizing a dosimetry of a radiation, comprising,
• an ionizing detector (1IC) configured for characterizing a dosimetry of a radiation beam (5) propagating along a Z-axis, the ionizing detector (1 IC) comprising a matrix of ionisation chambers (ICi) distributed over a plane (X,Y) normal to the Z-axis, wherein the ionizing detector has a first spatial resolution over the plane (X, Y),
• an additional detector (1A) different from the ionizing detector (1IC) and having a second spatial resolution over the plane (X, Y) higher than the first spatial resolution over the plane (X, Y) of the ionizing detector and is positioned in series along the Z-axis relative to the ionizing detector (1IC), and
• an intelligence (10) configured to calculate a distribution of calculated doses (Dij) from the doses (DICi) measured by the ionizing detector (1IC) and the doses (DAij, DA0j) measured by the additional detector (1A).

## Description

### FIELD OF THE INVENTION

The invention relates to the field of detectors for performing the dosimetry of a radiation, for example a beam of protons, electrons, helium ions, carbon ions, or oxygen ions, or beams of an electromagnetic radiation, such as X-ray or γ-ray. The detector of the present invention relies on the high accuracy and low beam energy dependence of the measures obtained with ionisation chambers with a substantially finer spatial resolution than available with ionisation chambers.

### BACKGROUND OF THE INVENTION

Radiation therapy for treating tumoural cells in a patient is widely used. Different radiations can be considered, including beams of protons, electrons, helium ions, carbon ions, or oxygen ions, or beams of an electromagnetic radiation, such as photons, preferably X-ray or *γ*-ray. A dose prescription is defined by a physician in the form of a treatment plan (TP) and a treatment plan system (TPS) optimises the treatment plan (TP) to deliver the prescribed doses at the prescribed regions.

Quality Assurance (QA) refers to a process that ensures quality of patient treatments, i.e., to guarantee accurate and safe treatments. The dosimetry chain requires Machine Quality Assurance and Patient Specific Quality Assurance (PSQA). The detector of the present invention is suitable for both machine and patient specific quality assurance and is preferably, but bot exclusively applied to patient specific quality assurance (PSQA). Quality assurance is needed to verify that the treatment delivery corresponds to the doses calculated by the TPS. For example, PSQA generally includes measuring dosimetric parameters of an emitted radiation in 2D or 3D. The most common form of PSQA involves the comparison of TPS dose calculations with 2D or 3D dose measurements. The gamma evaluation method is widely used to compare such measurements. Currently, two other forms of PSQA are more and more used in clinics: log-based QA and PSQA based on an independent dose calculation by Monte Carlo simulations.

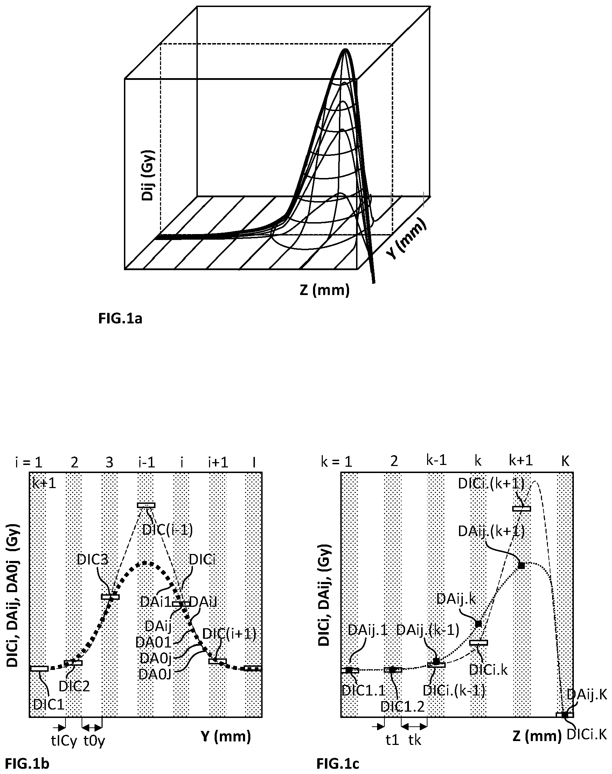

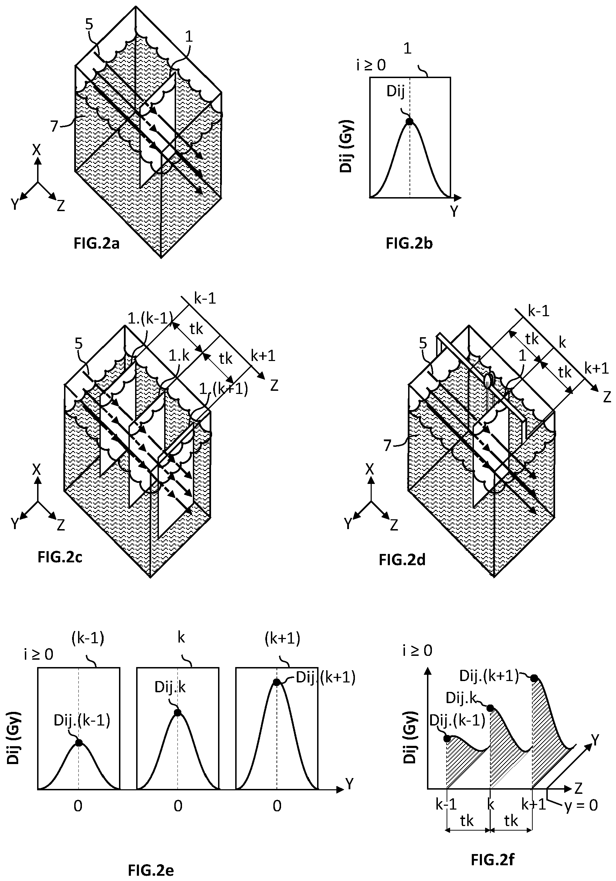

As illustrated in Figure 2a, a 2D-detector (1) is positioned across the path of a radiation beam (5) propagating along a Z-axis. A 2D-detector (1) measures a dose distribution on a "slice" in a plane (X, Y) normal to the Z-axis, as illustrated in Figures 1b and 2b. The 2D-detector can be positioned in a phantom (7) defining a water equivalent thickness (WET) corresponding to an equivalent depth in the patient's body. As shown in Figures 2a, 2c, and 2d, the phantom can be a tank filled with water or other liquid. It can also be made of plates of given thicknesses and given materials of known WET. A 3D-dose distribution as shown in Figure 1a can be obtained by positioning several 2D-detectors along the Z-axis as illustrated in Figure 2c, or by moving the 2D-detector (1) along the Z-axis, as shown in Figure 2d. The dose distributions over several slices is thus measured as shown in Figure 2e, and a dose distribution along the Z-axis can be obtained as shown in Figures 1c and 2f.

Various detectors are known for measuring the doses deposited by given radiations at a given WET. One of the most widely used detectors is an ionisation chamber detector (= IC-detector) formed by an array of ionisation chambers distributed on a plane (X, Y), such as described in EP1889281. As shown in Figures 6b and 6c, an ionization chamber (ICi) is composed of two polarization electrodes (3-, 3+) separated by a gap (3g) filled with a fluid, preferably a gas, generally air, or a liquid. The fluid is ionized by the radiation (5) traversing the ionization chamber (ICi) forming charged ions. An electric field is created by an external voltage source applied between the two polarization electrodes (3-, 3+). The charged ions are collected by the electrodes creating a measurable electric charge. For a given electric field applied to the ionization chamber, for a given design of the ionization chamber including the width of the gap, given dimensions of the electrodes (3-, 3+), etc., dosimetric parameters including the radiation dose and distribution of linear energy transfer (LET) can be determined from the amplitude of the charge measured at the electrodes (3-, 3+) by a measuring device (3V) (e.g., voltmeter or amperemeter).

lonisation chambers, however, provide a marginal spatial resolution of the order of the mm (e.g., 5 to 8 mm), which severely limits the precision of dose maps built from the measurement with IC-detectors. Increasing the spatial resolution of arrays of ionisation chambers has proven very difficult because the signal-to-noise ratio deteriorates quickly when the distance (tOx, t0y) separating adjacent ionisation chambers is reduced.

Other detectors are available on the market. For example, 2D semiconductor-based detectors are alternatives to IC detectors, offering substantially finer spatial resolution of the order of the µm (e.g., 10 to 300 µm). A semiconductor detector is a solid-state detector detecting and converting radiation to electric charges. This can be achieved by using either direct conversion or indirect conversion. In direct conversion detectors, the ionising radiation produces electron-hole pairs directly in the semiconductor. Under the influence of an electric field, free electrons and holes travel to the electrodes, where a current can be measured. Indirect conversion detectors include scintillating detectors, comprising a scintillation layer that converts the ionizing radiation into optical photons, converted into electrical charges by photodetectors. Certain semiconductor-based detectors can also be used to quantify linear energy transfer (LET) of particles. Thermoluminescent detectors are another type of indirect conversion detectors.

The values measured with both direct and indirect conversion semiconductor detectors, however, exhibit a high energy dependence and can "quench" when exposed to particle radiations. While the mechanism of ionization quenching is not fully understood, the result is a response of the detector deviating from the actual dose in regions with "high" linear energy transfer values. As shown in Figure 1c, this is the case for example in the region of the Bragg peak with clinical proton beams (compare in Figure 1c, the thin dashed Bragg peak measured with ionization chambers with the thick dotted line measured with a semiconductor detector subject to quenching).

In continuation, direct conversion detectors are referred to as *"semiconductor detectors"* and indirect conversion detectors are referred to as *"scintillating detectors"* or *"thermoluminescent detectors".*

There is a need to develop a new generation of dosimetry detectors dedicated to PSQA. This new generation of detector should give a fast and accurate response with a high spatial resolution under different conditions, including conventional dose rate and dose as well as ultrahigh dose rate and high dose (also called FLASH) irradiation, different types of particles, different delivery modalities (scattered beams, scanned pencil beams), different beam time structures (pulsed beams, continuous beams), and the like.

The present invention proposes a detector for characterizing a dosimetry of a radiation combining high accuracy, high precision and high spatial resolution, with a substantially lower energy dependence of the measured values. These and other advantages of the present invention are described in more detail in the following sections.

### SUMMARY OF THE INVENTION

The present invention concerns a detector for characterizing a dosimetry of a radiation, comprising an ionizing detector configured for characterizing the dosimetry of a radiation beam propagating along a Z-axis. The radiation beam can be a beam of charged particles, preferably protons, electrons, helium ions, carbon ions, or oxygen ions, or can be a beam of an electromagnetic radiation, preferably photons, more preferably X-ray or *γ*-ray.

The ionizing detector comprises a matrix of ionisation chambers distributed over a plane (X,Y) normal to the Z-axis. Each ionisation chamber comprises first and second electrode separated by a medium, wherein the ionizing detector has a first spatial resolution over the plane (X, Y).

An additional detector (1A) different from the ionizing detector and having a second spatial resolution over the plane (X, Y) finer than the first spatial resolution over the plane (X, Y) of the ionizing detector is positioned in series along the Z-axis relative to the ionizing detector. The detector comprises or is coupled to an intelligence configured to calculate a distribution of calculated doses from the doses measured by the ionizing detector and the doses measured by the additional detector.

The additional detector can be selected among a group consisting of a semiconductor detector, a scintillating detector, a thermoluminescent detector, a chemical detector including films, polymer gels, and alanine detectors.

The ionizing detector has a thickness measured along the Z-axis and the additional detector has a thickness measured along the Z-axis. The detector with the thickness yielding a lowest water equivalent thickness (WET) for a given radiation is preferably positioned upstream along the Z-axis relative to the radiation beam. For example, the additional detector can be a semiconductor detector positioned upstream of the ionizing detector along the Z-axis relative to the radiation beam. Alternatively, the additional detector can be a scintillating detector positioned downstream of the ionizing detector along the Z-axis relative to the radiation beam.

The detector has a thickness (t1) measured along the Z-axis which is preferably lower than 100 cm, more preferably lower than 70 cm, more preferably lower than 50 cm more preferably less than 10 cm, or less than 5 cm. Detectors (1) having a physical thickness (t1) in the upper range can for example comprise a scintillator detector as additional detector (1A), which comprises a thin scintillating plate, but also requires an optical structure including e.g., a mirror and photodetector such as a camera (e.g., a CCD camera) which considerably increases the thicknesses (1A, t1) of the additional detector (1A) and thus of the detector (1). Detectors having a physical thickness (t1) in the lower range can for example comprise a semiconductor detector as additional detector (1A). A distance (tIC-A) between the effective point of measurement of the ionizing detector and the effective point of measurement of the additional detector is preferably not more than 5 cm, preferably not more than 2 cm. In some embodiments, the IC-detector (1IC) and the additional detector (1A) can even contact each other, reducing the distance to close to 0 (i.e., tIC-A → 0). It is preferred that the thickness (t1) of the detector (1) corresponds to a WET of not more than 5 g / cm².

In a preferred embodiment, the ionisation chambers are distributed over the plane (X, Y) of the ionizing detector, which has a resolution of at least one ionisation chamber (IC) per cm², preferably at least 1.5 ionisation chambers (ICi) per cm². It is preferred that the additional detector has a pixel resolution of at least twice the resolution of the ionizing detector. The additional detector can have a resolution of at least 50 pixels / cm², or at least 70 pixels / cm², or at least 100 pixels / cm².

The intelligence is preferably configured to also determine a distribution of linear energy transfer (LET) of the radiation from the measured doses.

In a preferred embodiment, each ionisation chamber is separated from an adjacent ionisation chamber of the same ionizing detector by an inter-chamber space. One or more than one sensor (Aij) of the additional detector faces an ionisation chamber of the ionizing detector and one or more than one sensor of the additional detector faces an inter-chamber space. The sensors of the additional detector have a higher energy dependence than the ionisation chambers of the ionizing detector. The calculated doses, Dij, in a unit volume enclosed or intersecting the detector at the level of an ionisation chamber are a function Dij = f(DAij, DICi), of the dose, DICi, measured by the ionisation chamber and of the doses, DAij, measured by a given sensor of the additional detector facing the ionisation chamber along the Z-axis. Preferably the function Dij = f(DAij, DICi) also depends on one or more of
- doses, DICia, measured by neighbouring ionisation chambers adjacent to the unit volume,
- doses, DAija, measured by neighbouring sensors of the additional detector facing the ionisation chamber, and preferably those which are adjacent to the given sensor, and
- doses, DA0ja, measured by neighbouring sensors of the additional detector facing an inter-chamber space adjacent to the unit volume.

The calculated dose, Dij, can depend on a subfunction f1 (DICAij) relating the dose (DICi) measured by the ionisation chamber and the doses (DAij) measured by the sensors of the additional detector facing the ionisation chamber along the Z-axis (i.e., Dij = f(DAij, DICi, f1 (DCAij))). The subfunction f1 (DICAij) is preferably a ratio (DICi / DAij) or a difference (DICi - DAij) between the doses (DAij and DICi) measured by the additional detector and the ionizing detector.

In a preferred embodiment, at least one sensor of the additional detector faces an inter-chamber space. The calculated doses (DOj) at the level of a given sensor enclosed in an intermediate unit volume intersecting the detector at the inter-chamber space between adjacent ionisation chambers are a function, D0j = g(DA0j, DICia, DAija), of at least,
- the dose (DA0j) measured by the given sensor of the additional detector, and one or more of,
- doses (DICia) measured by the neighbouring ionisation chambers adjacent to the intermediate unit volume, and one or more of
- doses (DAOja) measured at least by the neighbouring sensors of the additional detector enclosed in the same intermediate volume and adjacent to the given sensor, and
- doses (DAija, i > 0) of the neighbouring sensors of the additional detector facing the neighbouring ionisation chambers.

The present invention also concerns a dosimetry characterisation unit configured for characterizing a dosimetry in planes (X, Y) of a radiation beam propagating over a Z-axis perpendicular to the plane (X, Y) at different positions (k = 1 to K, with K > 1) along the Z-axis, separated from one another by a distance (tk). The dosimetry characterisation unit comprises a detector as described supra positioned at a position (k) and either,
- further, comprises (K-1) adjacent detectors as defined supra, aligned with the detector along the Z-axis and located at respective positions (k + m; m ≠ 0), or
- the detector is configured to be moved to the different positions.

In a preferred embodiment, the detector and preferably, the adjacent detectors are as described supra, wherein the calculated doses (Dij, D0j) in a unit volume enclosed or intersecting the detector at the level of an ionisation chamber (ICi) are a function Dij = f(DAij, DICi) and, preferably, a function, D0j = g(DA0j, DICia, DAija). The functions Dij = f(DAij, DICi) and / or D0j = g(DA0j, DICia, DAija) determined at the position (k), are / is also a function of one or more of,
- the doses (DAija) measured by the corresponding sensors at positions ((k-1); (k+1)) directly adjacent to the position (k), and / or
- the doses (DAOja) measured by the corresponding sensors at positions ((k-1); (k+1)) directly adjacent to the position (k).

The present invention also concerns a method for characterizing a dosimetry of a radiation beam propagating along a Z-axis, comprising,
- positioning perpendicular to the Z-axis a dose detector as defined supra,
- propagating a radiation beam along the Z-axis crossing the dose detector,
- measuring the doses (DICi, DAij, DA0j) with the ionizing detector and the additional detector of the dose detector,
- calculating the calculated doses (Dij, D0j) as a function of the thus measured doses (DICi, DAij, DA0j).

### SHORT DESCRIPTION OF THE DRAWINGS

These and further aspects of the invention will be explained in greater detail by way of example and with reference to the accompanying drawings in which:
**Figures 1a** shows a 3D-representation) of a dose deposition in a plane (Y, Z) with a Bragg peak typical of a proton beam dose deposition.
**Figures 1b** shows a 2D-dose deposition on the Y-axis normal to the Z-axis, at the level of the Bragg peak of Figure 1a, measured with an array of ionisation chambers (= thin dashed line) and with semiconductor detectors (thick dotted line). The shaded columns represent the positions of the various ionisation chambers (ICi) with i = 1 to I.
**Figure 1c** shows a 2D-dose deposition on the Z-axis, measured by a series of detectors positioned along the Z-axis, with arrays of ionisation chambers (= thin dashed line) and with semiconductor detectors (thin dotted line). The shaded columns represent the positions of the various detectors with k = 1 to K.
**Figure 2a** shows a detector positioned in a phantom and intersecting the trajectory of a radiation beam.
**Figure 2b** shows the dose distribution along the Y-axis measured by the detector of Figure 2a.
**Figure 2c** shows a series of detectors aligned along the Z-axis in a phantom, intercepting a radiation beam.
**Figure 2d** shows a detector movingly positioned along the Z-axis in a phantom and intersecting the trajectory of a radiation beam.
**Figure 2e** shows the dose distributions along the Y-axis measured by the three detectors of Figure 2c or by the detector of Figure 2d at three different positions.
**Figure 2f** plots the doses Dij.(k-1), Dij.k, Dij.(k+1) of the dose distributions of Figure 2e as a function of the position of the detector along the Z-axis.
**Figures 3a & 3b** shows (a) a series of additional (semiconductor) detectors and (b) the corresponding proton beam 3D-dose deposition plot obtained therewith, with quenching effect visible by the squat geometry of the Bragg peak.
**Figures 4a & 4b** shows (a) a series of ionising chambers detectors and (b) the corresponding proton beam 3D-dose deposition plot obtained therewith, with low spatial resolution as shown by the shaded squares.
**Figures 5a and 5b** shows (a) a series of detectors according to the present invention formed by an additional detector as in Figure 3a associated in series with an ionisation chambers detector as in Figure 4a, and (b) the corresponding proton beam 3D-dose deposition plot obtained therewith combining accuracy and high spatial resolution.
**Figures 6a** shows a detector according to the present invention formed by a semiconductor detector as in Figure 3a associated in series with an ionisation chambers detector as in Figure 4a.
**Figures 6b & 6c** shows two embodiments of ionisation chambers.
**Figures 6d** shows a front view (on a plane (X, Y)) of a detector according to the present invention showing the alignments of the sensors (Aij, A0j) relative to the ionisation chambers (ICi).
**Figure 7a** shows a deposited dose (DICi) measured by a single ionisation chamber (ICi) compared with the deposited doses (DAij) measured by the semiconductors facing the single ionisation chamber along the Z-axis and showing a quenching effect with lower values than measured by the ionisation chamber.
**Figure 7b** shows the calculated deposited doses (Dij) according to the present invention, from the single value measured by the ionisation chamber combined with the values measured by the semiconductor detector as shown in Figure 7a.
**Figure 8a** shows the three deposited doses (DICi) measured by three ionisation chambers (ICi) compared with the deposited doses (DAij) measured by the semiconductors facing each of the three ionisation chambers along the Z-axis and the deposited doses (DA0j) measured by the semiconductors facing an intermediate volume between adjacent ionisation chambers.
**Figure 8b** shows the calculated deposited doses (Dij) according to the present invention, from the three values measured by the three ionisation chambers combined with the values measured by the semiconductor detector as shown in Figure 8a.
**Figure 9a** shows the relationship between the various ionisation chambers (ICi) of the IC-detector (1IC) and the sensors (Aij, i > 0) of the additional detector (1A) facing an ionisation chamber (ICi) with the intelligence (10) to calculate the calculated dose (Dij) corresponding to a given sensor (Aij, i > 0).
**Figure 9b** shows the relationship between the sensors (A0) of the additional detector (1A) facing a inter-chamber space and the neighbouring ionisation chambers (ICi) of the IC-detector (1IC) with the intelligence (10) to calculate the calculated dose (Dij) a corresponding to a given sensor (A0j-.

### DETAILED DESCRIPTION OF THE INVENTION

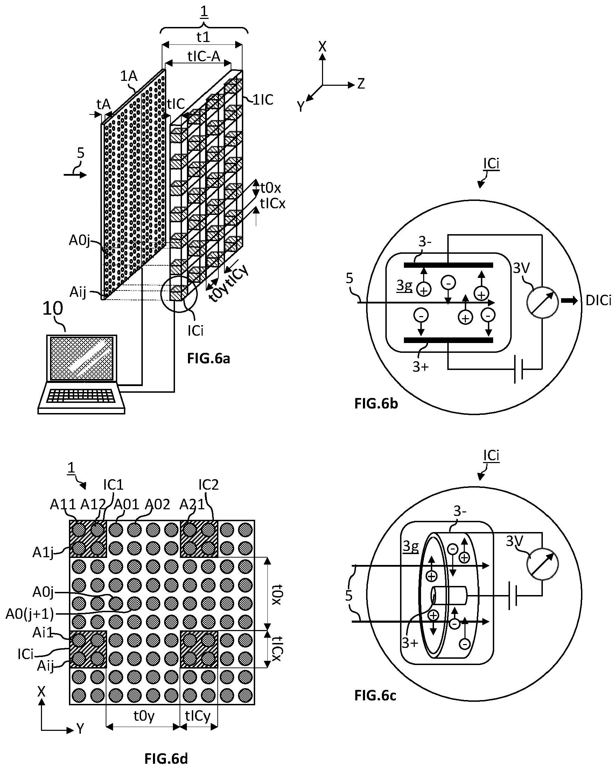

As illustrated in Figure 6a, the detector (1) for characterizing a dosimetry of a radiation beam (5) according to the present invention comprises an ionizing detector (1 IC) and an additional detector (1A) aligned along a Z-axis, both configured for characterizing the dosimetry of the radiation beam (5) propagating along the Z-axis. The radiation beam (5) can be a beam of charged particles, preferably protons, electrons, helium ions, carbon ions, or oxygen ions, or is a beam of an electromagnetic radiation, preferably photons, preferably X-ray or *γ*-ray.

The ionizing detector (1IC) comprises a matrix of ionisation chambers (ICi) distributed over a plane (X,Y) normal to the Z-axis. As shown in Figures 6b and 6c, each ionisation chamber (Ici) comprises first and second electrodes (3-, 3+) separated by a medium, preferably a gas, such as air, or a liquid. The ionizing detector has a first spatial resolution over the plane (X, Y).

The additional detector (1A) is different from the ionizing detector (1IC) and has a second spatial resolution over the plane (X, Y) finer than the first spatial resolution over the plane (X, Y), of the ionizing detector (Ici). The additional detector is positioned in series along the Z-axis relative to the ionizing detector (1IC).

The detector also comprises or is coupled to an intelligence configured to calculate a distribution of calculated doses (Dij) from the doses (DICi) measured by the ionizing detector (1 IC) and the doses (Daij, DA0j) measured by the additional detector (1A).

### DETECTOR CONFIGURATION FOR QUALITY ASSURANCE

As shown in Figures 1b, 2a and 2b, a detector (1) for quality assurance, preferably for PSQA according to the present invention is a 2D-detector configured for characterizing a dosimetry of a radiation beam (5) in a plane (X, Y) normal to the radiation beam propagating along a Z-axis. The desired depth in a patient's body of the plane (X, Y) can be simulated by positioning the detector (1) in a phantom (7) at a corresponding water equivalent thickness (WET). A phantom is generally formed by a tank filled with water. It can also be filled with a different liquid or the phantom can be formed of solid plates of known WET and given thicknesses. The detector (1) is therefore positioned at a strategic depth or WET of interest. For example, for proton beams, the plane (X, Y) preferably intercepts the Bragg curve.

As shown in Figures 1a, 1c, and 2c to 2f, a 3D-dose distribution map can be obtained by combining the dose distributions along various planes (X, Y) distributed along the Z-axis. This can be obtained with a dosimetry characterization unit as shown in Figure 2c by aligning a number of detectors (1) along the Z-axis or by moving a single detector along the Z-axis, e.g., along a rail as shown in Figure 2d.

The detector configurations described supra and illustrated in Figures 2a to 2f require, however, that the detectors used show a high enough spatial resolution and a high enough accuracy, precision, and reliability. In particular, the values measured with the detectors shall not be dependent on quenching effects. As discussed in the section "Background of the Invention" supra, however, this combination is not satisfactorily met by the ionisation chamber based detectors currently available on the market. This is achieved with the detector (1) of the present invention, comprising an ionisation detector (1IC) and an additional detector (1A) aligned in series along the Z-axis.

### IONISATION DETECTOR (1IC)

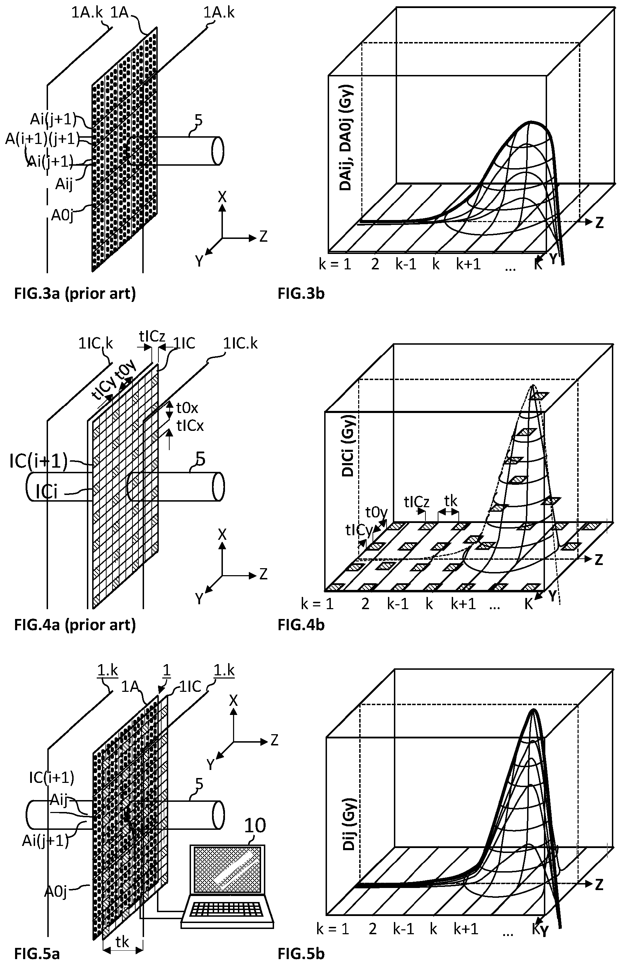

As shown in Figures 4a and 6a, the ionisation detector (= IC-detector) is formed by an array of ionisation chambers (ICi) distributed on the plane (X, Y). The ionisation chambers (ICi) comprise first and second electrodes (3-, 3+) separated by a medium, preferably a gas such as air or a liquid. As shown in Figures 6b and 6c the electrodes (3-, 3+) can be flat electrodes parallel to one another or can be concentric cylindrical electrodes as shown in Figures 6b and 6c. The current generated between the electrodes by the ionised molecules of the medium traversed by the radiation beam (5) is measured by a measuring unit (3V), such as an electrometer.

Each ionisation cell (ICi) has a width (tICy) and a height (tICx) measured along the Y- and X-axes, respectively (cf. Figures 6a and 6d). The physical thickness along the Z-axis of each ionisation cell (ICi) is not as important as the WET thereof. It is preferred that the WET of the ionisation cells be as low as possible, i.e., it is preferred that the radiation beam (5) loses as little energy as possible upon traversing an ionisation chamber (ICi). This is particularly important if the ionisation detector (1IC) is positioned upstream of the additional detector (1A). It is also important in case several detectors (1.k) are aligned in series along the Z-axis as shown in Figure 2c to establish a 3D-dose deposition mapping of the radiation beam (5).

The ionisation chambers (ICi) are separated from one another by distances (tOx, t0y) along the X- and Y-axes, respectively. The spatial resolution of the IC-detector (1IC) depends on the one hand, on the dimensions (tICx, tlCy) of the ionisation chambers (ICi) and, on the other hand, on the distances (tOx, t0y) separating adjacent IC-chambers (ICi). The response of an ionsation chamber must be corrected for well-known effects (temperature, pressure, polarity, recombination) in manners well known to a person skilled in the art. The dimensions of the ionisation chambers (ICi) is limited physically and technically. Reducing the distance (tOx, t0y) separating adjacent ionisation chambers is limited by the resulting quick deterioration of the signal-to-noise ratio. Consequently, the spatial resolution of an IC-detector (1IC) is limited and can currently not be increased substantially. It is preferred that the ionisation chambers (ICi) are distributed over the plane (X, Y) of the ionizing detector (1IC) which has a resolution of at least one ionisation chamber (IC) per cm², preferably at least 1.5 ionisation chambers (ICi) per cm². It is also preferred that the additional detector (1A) has a pixel resolution of at least twice the resolution of the ionizing detector (1IC).

The values (DICi) measured by IC-detectors (1IC, 1IC.k) are, however, very reliable and are not sensitive to quenching. Figure 4a shows a series of several IC-detectors (1IC) distributed along and perpendicular to the Z-axis and intersecting a radiation beam (5). Figure 4b shows the dose values measured by the various IC-detectors of Figure 4a aligned along the Z-axis obtained by combining the dose values measured by each IC-detector (1IC) of the series of IC-detectors of Figure 3a at a same position along the X-axis. The values measured by each ionisation cell (ICi) of each IC-detector (1IC) intersecting the (Y, Z) plane are represented by the shaded squares distributed on the (Y, Z) plane over the expected Bragg curve, with their thickness (tICz) measured along the Z-axis. It can be seen that the dose values (DICi) measured by each ionisation cell (ICi) fits the expected beam shape in each (X, Y) plane as illustrated in Figure 1b (thin dashed line), but the spatial resolution is insufficient to satisfactorily characterise the beam. The IC-detector (1IC) has a first spatial resolution over the plane (X, Y) which is of the order of the mm (e.g., 5 to 8 mm),

### ADDITIONAL DETECTOR (1A)

The additional detector (1A) must be different from the IC-detector (1IC) and must have a second spatial resolution over the plane (X, Y) finer than the first spatial resolution over the plane (X, Y) of the IC-detector (1IC). The additional detector (1A) preferably has a pixel resolution of at least twice the resolution of the ionizing detector (1IC). For example, the additional detector (1A) can have a resolution of at least 50 pixels / cm², preferably at least 100 pixels / cm².

The additional detector (1A) can be selected among a group consisting of a semiconductor detector, a scintillating detector, a thermoluminescent detector, a chemical detector including films, polymer gels, and alanine detectors. The additional detector (1A) is preferably a semiconductor detector or a scintillating detector.

Figure 3a shows an example of a series of additional detectors (1A, 1A.k) aligned along and perpendicular to the Z-axis and traversed by a radiation beam (5). Each additional detector (1A) is formed by an array of sensors (Aij, A0j) distributed over the corresponding plane (X, Y). As can be seen in Figure 6d, the dimensions of the sensors (Aij, A0j) are substantially smaller than the dimensions of the ionisation chambers (ICi) of the IC-detector (1 IC) and the sensors of the additional detector are separated from one another by a distance substantially smaller than the ionisation chambers (ICi) of the IC-detector (1IC). This results in a substantially denser array of sensors increasing the spatial resolution accordingly. Many additional detectors (1A), including semiconductor detectors are, however, sensitive to quenching when traversed by high-energy particle radiation beams (5). This can be the case, for example, in the region of the Bragg peak for proton beams. In the embodiment illustrated in Figure 3a, the additional detectors (1A) are semiconductor detectors and the sensors (Aij, A0j) are semiconductors.

Scintillating detectors comprise an array of scintillating layers. In response to an incident radiation beam (5), each scintillating layer generates photons in amounts dependent on the dose deposited by the radiation beam. These are collected by photodetectors (e.g., a camera) converting the optical energy into electrical energy whose magnitude can be measured.

Figure 3b shows the dose distribution over the plane (Y, Z) obtained by combining the dose values measured by each semiconductor detector (1A) of the series of semiconductor detectors of Figure 3a at a same position along the X-axis. Unlike the IC-detectors results plotted in Figure 4b, the spatial resolution of the dose distribution plotted in Figure 3b and measured with the semiconductor (additional) detectors (1A, 1A.k) of Figure 3a is high, with a second spatial resolution of the order of the µm (e.g., 10 to 300 µm). The absolute values (DAij, DA0j) obtained with the additional detectors of Figure 3a, however, are not reliable because of quenching effects observed at high energies, with a Bragg peak having a flattened geometry.

Figures 1b, 3b and 4b show that neither an IC-detector (1 IC) nor an additional detector (1A) (e.g., a semiconductor detector) satisfactorily characterizes the dosimetry of a radiation beam (5), either for poor spatial resolution or for poor reliability of the results at high energy particle beams. The detector (1) of the present invention solves this problem by combining the advantages of both detectors and compensating their respective drawbacks.

### DETECTOR (1) OF THE INVENTION = COMBINATION OF AN IC-DETECTOR (1IC) AND AN ADDITIONAL DETECTOR (1A)

Since all existing detectors have advantages but also unsatisfactory drawbacks, the present invention combines the advantages of different detectors to decrease and even neutralise their corresponding drawbacks. The detector (1) of the present invention comprises an IC-detector (1IC) having the first spatial resolution, with an additional detector (1A) positioned in series along the Z-axis and having the second spatial resolution finer than the first spatial resolution. Figure 6a shows an example of a detector (1) according to the present invention comprising the IC-detector (1IC) and the additional detector (1A), which is a semiconductor detector in this embodiment. The two detectors (1IC, 1A) are coupled in series along the Z-axis, separated from one another by a distance (tIC-A). For example, the distance (tIC-A) between the effective point of measurement of the ionizing detector (1IC) and the effective point of measurement of the additional detector (1A) is preferably not more than 5 cm, preferably corresponding to a WET of not more than 5 g / cm².

The additional detector (1A) has a physical thickness (tA) and the IC-detector (1IC) has a physical thickness (tIC) so that the detector has a physical thickness (t1) measured along the Z-axis of t1 = tIC + tA + (tIC-A). For example, the detector (1) can have a physical thickness (t1) measured along the Z-axis lower than 100 cm as discussed supra.

Among the IC-detector (1IC) and the additional detector (1A), the one having the physical thickness (tIC, tA) yielding a lowest water equivalent thickness (WET) for a given radiation is preferably positioned upstream along the Z-axis relative to the radiation beam (5), as it has the lowest energy absorption effect on the traversing radiation beam (5) before it reaches the second detector positioned downstream. For example, as illustrated in Figure 6a, the additional detector (1A) can be a semiconductor detector positioned upstream of the ionizing detector (1IC) along the Z-axis relative to the radiation beam (5). Alternatively, the additional detector (1A) can be a scintillating detector positioned downstream of the ionizing detector (1 IC) along the Z-axis relative to the radiation beam (5). The scintillating detector alone may have a lower WET than the IC-detector (1IC), but scintillating detectors are generally equipped with optical elements, including mirrors and cameras or photodetectors that can considerably increase the corresponding WET.

The detector (1) of the present invention measures the doses (DICi) with the IC-detector (1IC) and measures the doses (DAij, DA0j) with the additional detector (1A). The doses (DAij) are measured by the sensors (Aij) facing directly a corresponding ionisation cell (ICi) of the IC-detector (1IC) and the doses (DA0j) are measured by the sensors (AOj) facing directly an inter-chamber space separating adjacent ionisation cells (ICi) of the IC-detector (1IC). Figure 6d illustrates in a front view of the detector (1), the positions of the sensors (Aij, A0j) of the additional detector (1A) relative to the ionisation chambers (ICi) of the IC-detector (1IC). The intelligence (10) collects and uses the dose values (DICi, DAij, DA0j) measured by the IC-detector (1IC) and the additional detector (1A) forming the detector (1) of the present invention to determine a calculated dose distribution (Dij, D0j) having the accuracy, precision and reliability of the IC-detector and the second spatial resolution of the additional detector (1A).

Figure 5b shows the calculated dose distribution over the plane (Y, Z) measured with a series of detectors (1, 1.k) as illustrated in Figure 5a and calculated with the intelligence (10). The detector (1) is formed of a semiconductor detector as illustrated in Figure 3a aligned upstream of an IC-detector (1IC) as illustrated in Figure 4a. The calculated dose distribution (Dij = f(DAij, DA0j, DICi)) illustrated in Figure 5b characterizes the Bragg curve of the proton beam (5) of Figure 5a with high reliability, precision and accuracy, and with a high spatial resolution. The intelligence (10) is preferably also configured for calculating other dosimetric parameters from the measured doses (DICi, DAij, DA0j), such as the distribution of linear energy transfer (LET) of the radiation.

### INTELLIGENCE (10) AND CALCULATED DOSE DISTRIBUTION (Dij)

As shown in Figures 5a and 6a, the detector (1) of the present invention comprises or is coupled to an intelligence (10), represented in the Figures as a laptop, but it can take any other shape and format known in the art, such as an integrated circuit attached or plugged to the detector (1). The intelligence (10) is configured to calculate a distribution of calculated doses (Dij, D0j) from the doses (DICi) measured by the ionizing detector (1IC) and the doses (DAij, DA0j) measured by the additional detector (1A).

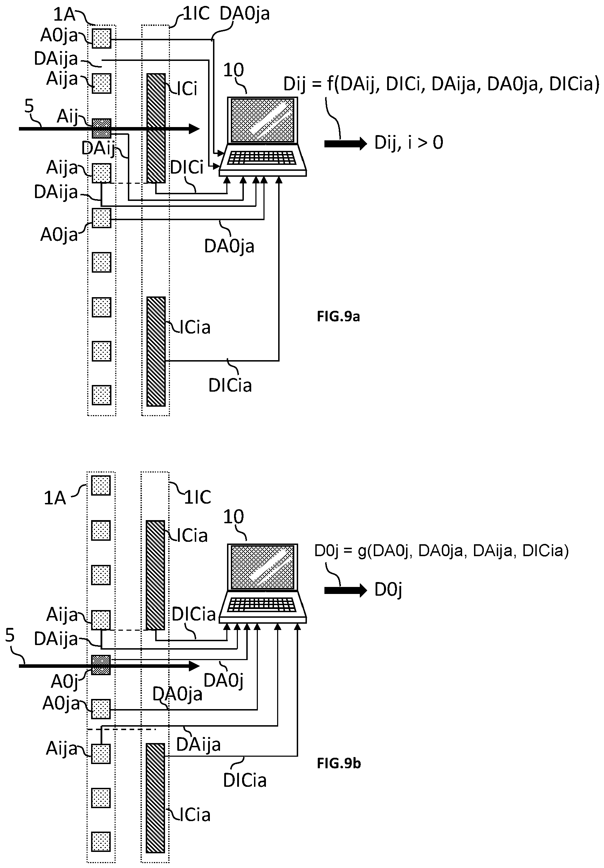

As shown in Figures 6d and 9a, at least one, preferably more than one sensor (Aij) of the additional detector (1A) faces an ionisation chamber (ICi) and at least one, preferably more than one sensor (AOj) of the additional detector (1A) faces an inter-chamber space. The additional detector (1A) therefore has a higher number of sensors (Aij, A0j) than the number of ionisation chambers (ICi) of the IC-detectors, thus conferring to the additional detector a second spatial resolution finer than the first spatial resolution of the IC-detector.

The sensors (Aij) are identical to the sensors (AOj) and the differing nomenclature refers to their positions relative to the ionisation chambers (ICi) only. The index "i" refers to the corresponding ionisation chamber (ICi) faced by a sensor (Aij) and the index "0" indicates that the sensor faces an inter chamber space. The index "j" counts the sensors as a function of their positions relative to the ionisation chambers (ICi). For example, in Figure 6d, four sensors (Aij) face each ionisation chamber (ICi), which are numbered Ai1, Ai2, Ai3, Ai4 (not all sensors are labelled in Figure 6d for sake of clarity). Similarly, the sensors (AOj) facing inter chamber spaces are numbered A01, A02, ..., A0j, ... To account for the fact that the sensors (Aij) are not different from the sensors (AOj), the following nomenclature is used in continuation:
- ICi, DICi, refer to the i^{th} ionisation chamber and the dose measured therewith. The index "i" in ICi and DICi is never equal to zero, i.e., when ICi and DICi are cited, necessarily i > 0, without need of specifying it.
- Aij, DAij, with i ≥ 0 refers to all the sensors of the additional detector (1A) and doses measured therewith, wherein
   ∘ the sensors Aij with i > 0 face a corresponding ionisation chamber (ICi) and
   ∘ the sensors Aij with i = 0 face an inter chamber space.
   When referring to Aij or DAij it is therefore necessary to specify whether i > 0, thus excluding A0j and DA0j, or i ≥ 0, therefore including A0j and DA0j.
- Dij, with i ≥ 0 refers to the calculated doses calculated from the values of DICi and DAij, wherein,
   ∘ Dij with i > 0 refers to a value calculated from a dose measured by a sensor Aij with i > 0 and
   ∘ Dij with i = 0 refers to a value calculated from a dose measured by a sensor Aij with i = 0.
   When referring to Dij it is therefore necessary to specify whether i > 0, indicating that Dij was calculated for a sensor Aij with i > 0 'i.e., excluding A0j, or with i ≥ 0, therefore indicating that Dij was calculated for a sensor Aij with i ≥ 0 'i.e., all sensors including A0j.

The sensors (Aij, i ≥ 0) of the additional detector have a higher energy dependence than the ionisation chambers (ICi) of the ionizing detector (1IC) and can therefore not safely be used alone to determine a dose as the values measured therewith are not entirely reliable in case the additional detector (1A) quenched due to exposure to radiation beams (5) having too high energies.

### Determination of a calculated dose, Dij, i > 0

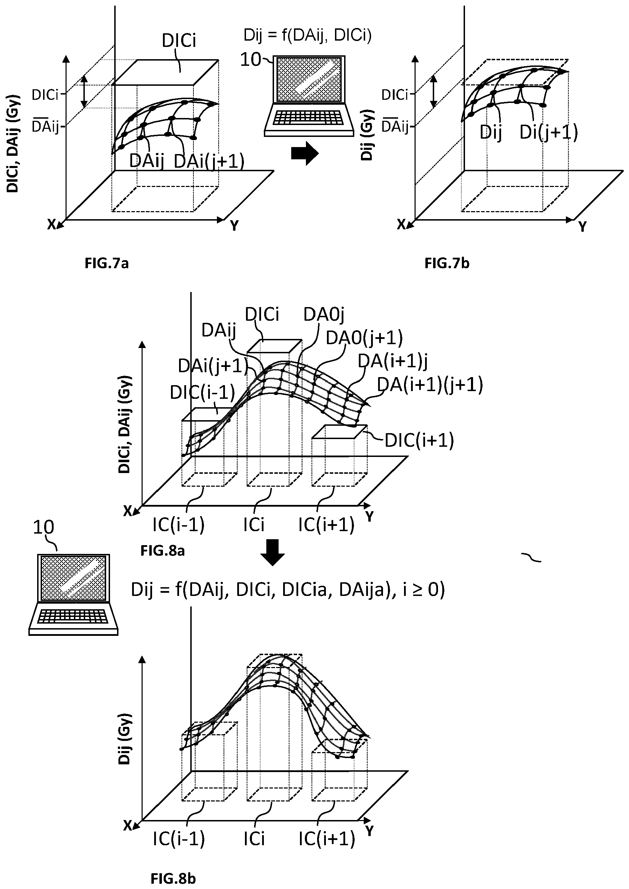

The calculated doses (Dij, i > 0) in a unit volume intersecting the detector (1) at the level of an ionisation chamber (ICi) can be a function Dij = f(DAij, DICi) with i > 0, of the dose (DICi) measured by the ionisation chamber (ICi) and of the doses (DAij, i > 0) measured by a given sensor (Aij, i > 0) of the additional detector (1A) facing the ionisation chamber (ICi) along the Z-axis. This is illustrated in Figures 7a and 7b. Figure 7a shows the single dose (DICi) measured by one ionisation chamber (ICi) over the area of the ionisation chamber (ICi) in the plane (X, Y). Figure 7a also shows the various dose values (DAij, with i > 0) measured by the sensors (Aij, i > 0) facing the ionisation chamber (ICi). It can be seen that the additional detector (1A) has a higher spatial resolution than the IC-detector (1IC). The dose values (DAij, i > 0) measured by the sensors (Aij, i > 0) of the additional detector (1A) are, however, lower than the single value (DICi) measured by the ionisation chamber (ICi) of the IC-detector (1IC), indicating that the additional detector (1A) suffered of a quenching effect, rendering the dose values (DAij, i > 0) unreliable.

Figure 7b shows that with the calculated doses (Dij, i > 0) the dose distribution over the area of the ionisation chamber (ICi) can be characterized with accuracy, precision and high spatial resolution, by calculating the dose (Dij, i > 0) as a function of the doses (DAij, i > 0) measured by the additional detector (1A) corrected to fit the dose (DICi) measured by the IC-detector (1IC).

The function f(DICi, DAij) for determining the values of the calculated doses (Dij, i > 0) can take multiple forms and the present invention is not restricted to a single function. Essential is that the calculated dose (Dij, i > 0) is calculated on the basis of at least the doses (DICi) measured by the IC-detector (1 IC) and the doses (DAij, i > 0) measured by the additional detector (1A). For example, the calculated dose (Dij, i > 0) can depend on a subfunction f1 (DICAij) relating the dose (DICi) measured by the ionisation chamber (ICi) and the doses (DAij, i > 0) measured by the sensors (Aij, i > 0) of the additional detector facing the ionisation chamber (ICi) along the Z-axis (i.e., Dij = f(DAij, DICi, f1 (DCAij)), with i > 0). The subfunction f1 (DICAij) can be a ratio (DICi / DAij) or a difference (DICi - DAij) between the doses (DAij and DICi) measured by the additional detector (1A) and the ionizing detector (1IC).

For example and as shown in Figures 7a and 7b, the function f1 (DCAij) can be a difference (f1 (DCAij) = DICi - D̅A̅ij, with i > 0) between the dose (DICi) measured by the ionisation chamber (ICi) and an average dose (D̅A̅ij, i > 0), measured by the sensors (Aij, i > 0) of the additional detector (1A) facing the ionisation chamber (ICi). The calculated dose (Dij) could then be Dij = DAij + f1 (DCAij) = DAij + DICi - D̅A̅ij, with i > 0. Using the average dose (D̅A̅ij, i > 0), measured by the sensors (Aij, i > 0) of the additional detector (1A) facing the ionisation chamber (ICi) makes sense since the single dose value (DICi) measured by the ionisation chamber (ICi) is a value averaged over the area (tlCx × tlCy) of the ionisation cell (ICi).

It is clear that in case no quenching occurred on the additional detector (1A) during a-QA-measurement the subfunction f1 (DAICij) = 0 and no correction of the values (DAij, i ≥ 0) measured by the sensors (Aij, i ≥ 0) of the additional detector (1A) is required.

In an alternative example, the function f1 (DCAij) can be a ratio (f1 (DICi) = DICi / D̅A̅ij, with i > 0) of the dose (DICi) measured by the ionisation chamber (ICi) to an average dose (D̅A̅ij, i > 0), measured by the sensors (Aij, i > 0) of the additional detector (1A) facing the ionisation chamber (ICi). The calculated dose (Dij, i > 0) could then be expressed as Dij = DAij × f1 (DCAij) = DAij × DICi / D̅A̅ij, with i > 0. Other forms of the functions f(Aij, DICi, f1 (DCAij)) and f1 (DCAij) are of course possible, which are well within the competence and reach of the person skilled in the art.

In a preferred embodiment illustrated in Figure 9a, the calculated dose (Dij, i > 0) also depends on one or more of the following measured doses,
- doses (DICia) measured by the ionisation chambers (ICia) adjacent to the unit volume, and / or
- doses (DAija, i ≥ 0) measured by the sensors (Aija, i ≥ 0) of the additional detector (1A) neighbouring the given sensor (Aij), including,
   ∘ the doses (DAija, i > 0) measured by the neighbouring sensors (Aija, i > 0) facing the ionisation chamber (ICi), preferably at least by the neighbouring sensors directly adjacent to the given sensor (Aij), and / or
   ∘ the doses (DAOja) measured by neighbouring sensors (AOj) facing the inter-chamber spaces adjacent to the unit volume.

Taking into account the doses (DAija, DICia) measured by the neighbouring sensors (Aija) and / or neighbouring ionisation chambers (ICia) allows yielding a continuous, preferably smooth dose distribution curve on the plane (X, Y). For example, a condition of having a continuous derivative between the calculated dose (Dij) issued from the given sensor (Aij) and the calculated doses (Dija) issued from the neighbouring sensors (Aija) and neighbouring ionisation chambers (ICia).

It is clear that the function f1 (DCAij) can also be applied taking into account the doses (DICia) measured by the neighbouring ionisation chambers (ICi) and / or the doses (DAij, i ≥ 0) measured by the neighbouring sensors (Aij, i ≥ 0) of the additional detector (1A).

Figures 8a and 8b illustrate schematically a determination of the calculated doses (Dij, i ≥ 0), wherein for each sensor (Aij, i ≥ 0), beside the doses (DICi, DAij) measured by the corresponding ionisation chamber (ICi) and sensor (Aij), the calculated dose (Dij) takes also into account,
- the doses (DICia) including the doses (DIC(i-1), DIC(i+1)) measured by the neighbouring ionisation chambers (IC(i-1), IC(i+1)) and
- the doses (DAija, i ≥ 0) including the doses (DAij, DAi(j+1), DA(i+1)j, DAi(i+1)(j+1), DA0j, DA0(j+1), etc.) measured by the neighbouring sensors (Aija, i ≥ 0) including the sensors (Aij, Ai(j+1)j, A(i+1)j, Ai(i+1)(j+1), A0j, A0(j+1), etc.) (not shown),

Taking account of the signals of the neighbouring sensors (Aija) and / or neighbouring ionisation chambers (ICia) for the determination of the calculated values of the doses (Dij) ensures that the calculated dose distribution over the plane (X, Y) is continuous and preferably smooth.

### Determination of a calculated dose, D0j

The determination of the calculated dose (D0j) in an intermediate unit volume intersecting the detector (1) at the level of an inter-chamber space between neighbouring ionisation chambers (ICia) adjacent to the inter-chamber space can be carried out as follows. In a first embodiment, the calculated doses (DOj) in an inter chamber space can simply be interpolated between the calculated doses (Dij, i > 0) of two or more adjacent ionisation chambers (ICi) surrounding the inter chamber space.

In a preferred embodiment, the calculated dose (DOj) in an intermediate unit volume are determined as follows. First, the additional detector (1) must comprise at least one, preferably more than one sensor (AOj) facing an inter-chamber space of the IC-detector (1IC). As illustrated in Figure 9b, the calculated doses (DOj) at a level of a given sensor (AOj) enclosed in the intermediate unit volume can be a function, D0j = g(DA0j, DICia, DA0ja, DAija), of at least,
- the doses (DA0j) measured by the given sensor (AOj) of the additional detector (1A),
- the doses (DICia) measured by the neighbouring ionisation chambers (ICia) adjacent to the intermediate unit volume, and
   one or more of,
- the doses (DA0ja) measured at least by the neighbouring sensors (AOja) of the additional detector (1A) enclosed in the same intermediate volume and adjacent to the given sensor (AOj), and
- the doses (DAija, i > 0) of the neighbouring sensors (Aija, i > 0) of the additional detector (1A) facing the neighbouring ionisation chambers (ICia).

### High resolution 2D-dose distribution on plane (X, Y) and 3D dose distribution in volume (X, Y, Z)

A high resolution 2D-dose distribution on the plane (X, Y) by a radiation beam (5) as shown in Figure 1b (thin dashed line) can be obtained by determining the calculated doses (Dij, i ≥ 0) at the level of all sensors (Aij, i ≥ 0), including the sensors (Aij, i > 0) facing an ionization chamber (ICi) and the sensors (A0j) facing an inter-chamber space as discussed supra.

A high-resolution 3D-dose distribution map on a volume (Y, Z) at a given position along the X-axis as illustrated in Figures 1a and 5b can be obtained by establishing high resolution 2D-dose distribution maps at different positions (k) along the Z-axis, corresponding to different depths in the patient's body. This can be achieved with a dosimetry characterization unit comprising either, as shown in Figures 2c, a plurality of detectors (1.k) aligned at corresponding positions (k = 1 to K, K> 1) along the Z-axis or, as shown in Figure 2d, a single detector (1) configured for being moved to different positions (k = 1 to K, K > 1) along the Z-axis. In both cases, the dose distribution measured along the Z-axis are discrete corresponding to the discrete positions (k) of the single or plurality of detectors (1, 1.k) during the measurements. Aligning a plurality of detectors (1.k) as shown in Figure 2c has the advantage that the characterization can be performed with a single measurement run. The drawbacks are, on the one hand, that a plurality (K) of detectors (1.k, k = 1 to K) are required and, on the other hand, a fraction of the energy of the radiation beam (5) is released upon interaction with each of the successive detectors (1.k), which can flaw the values at higher depths along the Z-axis, depending on the number (K) of detectors used. Using a single detector (1) moved at different positions (k) along the Z-axis has the advantage of requiring a single detector (1). The drawback is that the several (K) measurements must be repeated, which take a longer time as the detector (1) must be moved to its new positions along the Z-axis and (2) must be reset prior to starting a next measurement run.

The dosimetry characterisation unit therefore comprises a detector (1) as discussed supra, positioned at a position (k) and either,
- further, comprises (K-1) adjacent detectors (1.k) same as the detector (1), aligned with the detector (1) along the Z-axis and located at respective positions (k + m; m ≠ 0), as shown in Figure 2c, or
- the detector (1) is configured to be moved to the different positions (k), as shown in Figure 2d. Figure 2d shows the detector (1) mounted on a rail, but a rail is not essential. It suffices that the detector (1) can be fixed at the different positions (k).

For determining the calculated dose distribution on a plane (Y, Z) as shown in Figures 1a and 5b, or the dose distribution along the Z-axis as shown in Figure 1c -which is nothing but the intersection of the surface in Figure 1a with the plane y = 0 represented with a dashed line in Figure 1a- the calculated dose distributions along the Y-axis can be determined for each position (k) independently from one another for each position (k), and the values plotted along the Z-axis. This is illustrated in Figure 2e, showing the calculated dose distributions along planes (X, Y) at different positions ((k-1), k, (k+1)) along the Z-axis. Figure 2f shows the alignment along the Z-axis at the respective positions (k) of the calculated dose distributions over the planes (X, Y) of Figure 2e (only half (y ≥ 0) of the dose distributions are shown for sake of clarity). By repeating this operation over the full range of the radiation beam (5), it is possible to create a high resolution calculated dose distribution map over the planes (X, Y) distributed along the Z-axis and separated by a distance (tk), as shown in Figures 1a and 5b.

In a preferred embodiment, rather than calculating the dose distributions over the planes (X, Y) for each position (k) independently of the other positions ((k-1), (k+1), etc.), the calculated dose distribution in a plane (X, Y) at a position (k) also takes into account the dose values measured with the adjacent detectors (1k) at adjacent positions ((k-1), (k+1)). This can be achieved by ensuring that the functions f(DAij, DICi) and / or D0j = g(DA0j, DICia, DAija), are / is a function of at least,
- the doses (DAija) measured by the corresponding sensors (Aija) at positions ((k-1); (k+1)) directly adjacent to the position (k), and / or
- the doses (DAOja) measured by the corresponding sensors (AOja) at positions ((k-1); (k+1)) directly adjacent to the position (k).

This allows a smoother dose distribution along the Z-axis to be obtained, with no or less steps between the calculated dose values (Dij, i ≥ 0) determined as various positions (k).

### METHOD FOR CHARACTERIZING A DOSIMETRY OF A RADIATION BEAM (5)

The present invention also concerns a method for characterizing a dosimetry of a radiation beam (5) propagating along a Z-axis, comprising,
- positioning perpendicular to the Z-axis a dose detector (1) as described supra,
- propagating a radiation beam (5) along the Z-axis crossing the dose detector (1),
- measuring the doses (DICi) with the ionizing detector (1IC) and the doses (DAij, i ≥ 0) with the additional detector (1A) of the dose detector (1),
- calculating the calculated doses (Dij, i ≥ 0) as a function of the thus measured doses (DICi, DAij, DA0j).

The detector (1) is preferably positioned in a phantom to form a dosimetry characterisation unit as illustrated in Figure 2a to control the WET build-up corresponding to the position (k) of the detector (1) along the Z-axis. The detector (1) can be configured for changing the positions of the plane (X, Y) to form a 3D dosimetry characterisation unit as illustrated in Figure 2c or can be accompanied by adjacent dosimetric dose detectors (1.k) distributed along the Z-axis to form a 3D dosimetry characterisation unit as illustrated in Figure 2b.

The detector (1), the dosimetry characterisation unit, and the method of the present invention allow reliable high resolution dosimetry mappings to be created, which are required for ensuring accurate QA with current radiation therapies as well as with new evolutions in radiation therapies, including FLASH therapy and adaptive therapy. In the framework of PSQA, the present detector permits to determine very rapidly a dose distribution, reducing PSQA operation time considerably. It also has the advantage of improving PSQA for small fields. This is achieved by combining the high accuracy, precision and reliability of the dosimetric values measured with IC-detectors (1IC) with the high resolution obtained with additional detectors (1A), such as semiconductor detectors and scintillating detectors. The gist of the invention is that when the advantages of the two types of detectors sum up, their disadvantages do not, with the disadvantages of one detector being compensated by the advantages of the other. This way, a synergistic effect is obtained, with the low spatial resolution of the IC-detector (1IC) being compensated by the high spatial resolution of the additional detector (1A) and with the high energy dependence of the additional detector (1A) being compensated by the high accuracy and precision of the values measured with the IC-detector (1IC). The intelligence (10) is essential to combine the values measured by each detector (1IC, 1A) to yield a calculated dose distribution (Dij, i ≥ 0) representative of reality.

| **#** | **Definition** |
|---|---|
| 1 | Detector |
| 1A | Additional detectror |
| 1IC | Ionizing detector |
| 1.k | Neighbouring detector |
| 3+, 3- | Electrodes of an ionisation chamber |
| 3g | Ionic gas in an ionisation chamber |
| 3V | Voltmeter of an ionisation chamber |
| 5 | Radiation beam |
| 10 | Intelligence |
| A0j | Sensor of the additional detector facing an inter-chamber space |
| Aij, i > 0 | Sensor of the additional detector facing an ionisation chamber ICi |
| DA0j | Dose measured by a sensor A0j |
| DAij, i>0 | Dose measured by a sensor Aij |
| D̅A̅ij | average dose measured by the sensors (Aij) facing the ionisation chamber (ICi) |
| DICi | Dose measured by an ionisation chamber ICi |
| D0j | Calculated dose in an intermediate unit volume |
| Dij | Calculated dose in a unit volume intercepting an ionisation chamber ICi |
| Dija | Calculated doses of the unit volumes adjacent to the intermediate unit volume |
| i>0 | Index of a given ionisation chamber and of sensors facing the given ionisation chamber |
| j | Index of the sensors |
| k= 1 to K | Positions of the plane (X, Y) along the Z-axis. |
| t0x | ionization chamber to ionization chamber distance along X-axis |
| t0y | ionization chamber to ionization chamber distance along Y-axis |
| t1 | Thickness of the dose detector along Z-axis |
| tA | Thickness of the additional detector along Z-axis |
| tlC | Thickness of the ionizing detector along Z-axis |
| tlCx | Size of the ionization cell along the X-axis |
| tICy | Size of the ionization cell along the Y-axis |
| tICz | Thickness of the IC-detector along the Z-axis |
| tk | Distance separating two adjacent measuring positions k |
| X, Y, Z | Reference axes |

## Claims

1. A detector (1) for characterizing a dosimetry of a radiation, comprising a ionizing detector (1IC) configured for characterizing a dosimetry of a radiation beam (5) propagating along a Z-axis, the ionizing detector (1IC) comprising a matrix of ionisation chambers (ICi) distributed over a plane (X,Y) normal to the Z-axis, each ionisation chamber (ICi) comprising first and second electrodes separated by a medium, wherein the ionizing detector has a first spatial resolution over the plane (X, Y),
**Characterized in that,** an additional detector (1A) different from the ionizing detector (1IC) and having a second spatial resolution over the plane (X, Y) finer than the first spatial resolution over the plane (X, Y) of the ionizing detector is positioned in series along the Z-axis relative to the ionizing detector (1IC), and
**in that** the detector comprises or is coupled to an intelligence (10) configured to calculate a distribution of calculated doses (Dij) from the doses (DICi) measured by the ionizing detector (1IC) and the doses (DAij, DA0j) measured by the additional detector (1A).

2. Detector according to claim 1, wherein the additional detector (1A) is selected among a group consisting of a semiconductor detector, a scintillating detector, a thermoluminescent detector, a chemical detector including films, polymer gels, and alanine detectors.

3. Detector according to claim 1 or 2, wherein the ionizing detector (1IC) has a physical thickness (tIC) measured along the Z-axis and the additional detector (1A) has a physical thickness (tA) measured along the Z-axis and wherein the one of the ionizing detector and additional detector having the physical thickness (tIC, tA) yielding a lowest water equivalent thickness (WET) for a given radiation is positioned upstream along the Z-axis relative to the radiation beam (5).

4. Detector according to claims 2 or 3, wherein,
• the additional detector (1A) is a semiconductor detector positioned upstream of the ionizing detector (1IC) along the Z-axis relative to the radiation beam (5) or
• the additional detector (1A) is a scintillating detector positioned downstream of the ionizing detector (1IC) along the Z-axis relative to the radiation beam (5).

5. Detector according to anyone of the preceding claims, wherein the radiation beam (5) is a beam of charged particles, preferably protons, electrons, helium ions, carbon ions, or oxygen ions, or is a beam of an electromagnetic radiation, preferably photons, preferably X-ray or *γ*-ray.

6. Detector according to anyone of the preceding claims, wherein the detector (1) has a physical thickness (t1) measured along the Z-axis lower than 100 cm.

7. Detector according to anyone of the preceding claims, wherein a distance (tIC-A) between the effective point of measurement of the ionizing detector (1IC) and the effective point of measurement of the additional detector (1A) is not more than 5 cm, preferably corresponding to a WET of not more than 5 g / cm².

8. Detector (1) according to anyone of the preceding claims, wherein,
• the ionisation chambers (ICi) are distributed over the plane (X, Y) of the ionizing detector (1IC) which has a resolution of at least one ionisation chamber (IC) per cm², preferably at least 1.5 ionisation chambers (ICi) per cm², and wherein
• the additional detector (1A) has a pixel resolution of at least twice the resolution of the ionizing detector (1IC).

9. Detector (1) according to anyone of the preceding claims, wherein the intelligence (10) is configured to also determine a distribution of linear energy transfer (LET) of the radiation from the measured doses (DICi, DAij, DA0j).

10. Detector according to anyone of the preceding claims, wherein,
• each ionisation chamber (ICi) is separated from an adjacent ionisation chamber of the same ionizing detector (1IC) by an inter-chamber space (tOx, t0y),
• one or more than one sensor (Aij) of the additional detector faces an ionisation chamber (ICi) of the ionizing detector (1IC),
• one or more than one sensor (AOj) of the additional detector faces an inter-chamber space,
• the sensors (Aij, A0j) of the additional detector have a higher energy dependence than the ionisation chambers (ICi) of the ionizing detector (1IC),
• the calculated doses (Dij) in a unit volume enclosed or intersecting the detector (1) at the level of an ionisation chamber (ICi) are a function Dij = f(DAij, DICi), of the dose (DICi) measured by the ionisation chamber (ICi) and of the doses (DAij) measured by a given sensor (Aij) of the additional detector facing the ionisation chamber (ICi) along the Z-axis, and preferably the function Dij = f(DAij, DICi) also depends on one or more of
∘ doses (DICia) measured by neighbouring ionisation chambers (ICia) adjacent to the unit volume,
∘ doses (DAija) measured by neighbouring sensors (Aija) of the additional detector (1A) facing the ionisation chamber (ICi), and preferably those which are adjacent to the given sensor (Aij), and
∘ doses (DAOja) measured by neighbouring sensors (AOja) of the additional detector (1A) facing an inter-chamber space adjacent to the unit volume.

11. Detector according to claim 10, wherein the calculated dose (Dij) depends on a subfunction f1(DICAij) relating the dose (DICi) measured by the ionisation chamber (ICi) and the doses (DAij) measured by the sensors (Aij) of the additional detector facing the ionisation chamber (ICi) along the Z-axis (i.e., Dij = f(DAij, DICi, f1(DCAij))), wherein the subfunction f1(DICAij) is preferably a ratio (DICi / DAij) or a difference (DICi - DAij) between the doses (DAij and DICi) measured by the additional detector (1A) and the ionizing detector (1IC).

12. Detector according to claim 10 or 11, wherein,
• at least one sensor (AOj) of the additional detector (1A) faces an inter-chamber space,
• the calculated doses (DOj) at the level of a given sensor (AOj) enclosed in an intermediate unit volume intersecting the detector (1) at the inter-chamber space between adjacent ionisation chambers (ICia) are a function, D0j = g(DA0j, DICia, DAija), of at least,
∘ the dose (DA0j) measured by the given sensor (AOj) of the additional detector (1A), and one or more of,
∘ doses (DICia) measured by the neighbouring ionisation chambers (ICia) adjacent to the intermediate unit volume, and one or more of
∘ doses (DAOja) measured at least by the neighbouring sensors (AOja) of the additional detector (1A) facing enclosed in the same intermediate volume and adjacent to the given sensor (AOj), and
∘ doses (DAija, i > 0) of the neighbouring sensors (Aija) of the additional detector (1A) facing the neighbouring ionisation chambers (ICia).

13. Dosimetry characterisation unit configured for characterizing a dosimetry in planes (X, Y) of a radiation beam propagating over a Z-axis perpendicular to the plane (X, Y) at different positions (k = 1 to K, with K > 1) along the Z-axis, separated from one another by a distance (tk), wherein the dosimetry characterisation unit comprises a detector (1) according to anyone of the preceding claims positioned at a position (k) and either,
• further, comprises (K-1) adjacent detectors (1.k) according to anyone of the preceding claims, aligned with the detector (1) along the Z-axis and located at respective positions (k + m; m ≠ 0), or
• the detector (1) is configured to be moved to the different positions (k).

14. Dosimetry characterisation unit according to the preceding claim, wherein the detector (1) and preferably, the adjacent detectors (1.k) are according to anyone of claims 10 to 12, and wherein the functions Dij = f(DAij, DICi) and / or D0j = g(DA0j, DICia, DAija) determined at the position (k), are / is also a function of one or more of,
• the doses (DAija) measured by the corresponding sensors (Aija) at positions ((k-1); (k+1)) directly adjacent to the position (k), and / or
• the doses (DAOja) measured by the corresponding sensors (AOja) at positions ((k-1); (k+1)) directly adjacent to the position (k).

15. Method for characterizing a dosimetry of a radiation beam (5) propagating along a Z-axis, comprising,
• positioning perpendicular to the Z-axis a dose detector (1) according to anyone of claims 1 to 12,
• propagating a radiation beam (5) along the Z-axis crossing the dose detector (1),
• measuring the doses (DICi, DAij, DA0j) with the ionizing detector (1IC) and the additional detector (1A) of the dose detector (1),
• calculating the calculated doses (Dij, D0j) as a function of the thus measured doses (DICi, DAij, DA0j).
